# EUROPÄISCHE PATENTSCHRIFT

(11) **EP 4 176 911 B1**
(45) Veröffentlichungstag und Bekanntmachung des Hinweises auf die Patenterteilung: **22.04.2026**
(21) Anmeldenummer: 22202646.0
(22) Anmeldetag: 20.10.2022
(51) Int. Cl.: A61M 1/14, A61M 1/16, A61M 1/34, G16H 20/40, G16H 40/63

(54) **MODULARE BLUTBEHANDLUNGSVORRICHTUNG**
MODULAR BLOOD TREATMENT DEVICE
DISPOSITIF MODULAIRE DE TRAITEMENT DU SANG

(30) Priorität: 08.11.2021 DE 102021129003
(43) Veröffentlichungstag der Anmeldung: 10.05.2023
(73) Patentinhaber: B. Braun Avitum AG, 34212 Melsungen (DE)
(72) Erfinder: JANIK, Waldemar, 34212 Melsungen (DE); LECKE, Tobias, 36179 Bebra (DE); WOLFF, Henrik, Dr., 34212 Melsungen-Adelshausen (DE); WÜRSCHMIDT, Tobias, 34346 Hann. Münden (DE)
(74) Vertreter: Winter, Brandl - Partnerschaft mbB

(56) Entgegenhaltungen:
- EP-A2- 0 997 157
- GB-A- 2 196 756
- US-A- 5 442 969
- US-A1- 2011 189 048
- US-A1- 2013 334 138
- US-A1- 2016 101 225

## Beschreibung

### Technisches Gebiet

Die vorliegende Erfindung bezieht sich auf eine modulare Blutbehandlungsvorrichtung gemäß Anspruch 1, insbesondere auf eine modulare Dialysevorrichtung, vorzugsweise eine modulare Hämodiafiltrationsvorrichtung, mit mehreren Modulen, die ausgebildet sind, zur Durchführung einer Blutbehandlung an einem Patienten lösbar, insbesondere mechanisch, elektrisch, elektronisch, fluidmechanisch und/oder hydraulisch, miteinander gekoppelt zu werden, einer Hauptsteuerungseinheit, die ausgebildet ist, die miteinander gekoppelten Module zur Durchführung der Blutbehandlung zu steuern, Sensoren und Aktoren.

### Stand der Technik

In der US 2011 / 0 189 048 A1 wird eine Blutbehandlungsvorrichtung in Form eines modularen Dialysesystems offenbart, welches ein Benutzerschnittstellenmodul, ein Wasserbehandlungsmodul und ein Dialysemodul aufweist. Gemäß US 2011 / 0 189 048 A1 wird ein herkömmliches Dialysesystem modularisiert, um dessen Transport zu erleichtern und so die Durchführung von Dialysebehandlungen abseits von Dialysezentren zu Hause zu vereinfachen. Im zusammengebauten Zustand entspricht das Dialysesystem gemäß US 2011 / 0 189 048 A1 einem herkömmlichen Dialysesystem, dessen Handhabung und Sicherheit an den Gebrauch durch Fachpersonal angepasst ist.

US 2013 / 0 334 138 A1 offenbart ein Blutbehandlungssystem mit einer Peritonealdialyseeinheit und einer Blutpumpeneinheit. Soll eine Peritonealdialyse durchgeführt werden, reicht es aus, lediglich die Peritonealdialyseeinheit zu verwenden. Soll eine Hämodialyse durchgeführt werden, wird die Blutpumpeneinheit an die Peritonealdialyseeinheit angeschlossen. Zur Steuerung der beiden Einheiten weist die Peritonealdialyseeinheit einen "master controller" auf, der mit einem "delegate controller" der Blutpumpeneinheit verbunden ist.

US 2016 / 0 101 225 A1 offenbart ein modulares Hämodialysesystem mit drei unterschiedlichen Typen von Modulen: Modul für Wasser, Modul für Dialysierflüssigkeit bzw. Dialysat und Therapiemodul. Das Modul für Wasser kann als Subsysteme ein Vorbehandlungssubsystem und einen Aktivkohlefilter aufweisen. Das Modul für Dialysierflüssigkeit bzw. Dialysat kann als Subsysteme ein Dialysierflüssigkeitssubsystem und ein Ultrafiltratsubsystem aufweisen. Das Therapiemodul kann als Subsysteme einen Dialysefilter, ein Blutbehandlungssubsystem sowie ein elektrisches bzw. softwaretechnisches Subsystem aufweisen. Ein Mechanisches Subsystem des Hämodialysesystems gemäß US 2016 / 0 101 225 A1 weist Bestandteile auf, die auf die drei Module (Waser, Dialysat, Therapie) aufgeteilt sind. Eine Hauptsteuerungseinheit ("controller") des Therapiemoduls steuert die Module für Wasser und für Dialysierflüssigkeit bzw. Dialysat.

EP 0 997 157 A2 offenbart ein Blutbehandlungsgerät mit einem "Blutsystem" und einem "Hydrauliksystem". Kontrolleinheiten des Blutbehandlungsgeräts sind miteinander über zwei Busse verbunden. Die Kontrolleinheiten gemäß EP 0 997 157 A2 weisen selbst keine Aktoren oder Sensoren auf.

Aufgabe der vorliegenden Offenbarung ist es deshalb, eine modulare Blutbehandlungsvorrichtung bereitzustellen, die eine verbesserte Sicherheit ermöglicht.

Diese Aufgabe wird durch Merkmale des Anspruchs 1 gelöst. Vorteilhafte Weiterbildungen sind Gegenstand der Unteransprüche.

### Zusammenfassung der Offenbarung

Eine offenbarungsgemäße modulare Blutbehandlungsvorrichtung weist mehrere Module, eine Hauptsteuerungseinheit, Sensoren und Aktoren auf. Die Module sind ausgebildet, zur Durchführung einer Blutbehandlung an einem Patienten lösbar miteinander gekoppelt zu werden.

"Gekoppelt" bedeutet insbesondere, dass eine mechanische, elektrische, elektronische, fluidmechanisch und/oder hydraulisch Verbindung besteht.

"Lösbar" bedeutet insbesondere, dass die miteinander gekoppelten Module zerstörungsfrei, vorzugsweise ohne Werkzeug, voneinander getrennt bzw. entkoppelt werden können.

Allgemein weist eine Blutbehandlungsvorrichtung einen Blutkreislauf auf. Vorzugsweise weist eine Blutbehandlungsvorrichtung zumindest einen weiteren Fluidkreislauf, beispielsweise einen Dialysierflüssigkeitskreislauf, auf. Bei einer Behandlung eines Patienten ist der Blutkreislauf über Anschlüsse mit dem Patienten verbunden. Im Rahmen der Offenbarung kann der Begriff Fluidsystem zur Verwendung kommen, wenn auf den Blutkreislauf und/oder den zumindest eine weiteren Fluidkreislauf Bezug genommen wird.

Die Hauptsteuerungseinheit ist ausgebildet die miteinander gekoppelten Module zur Durchführung der Blutbehandlung zu steuern.

Die Sensoren können beispielsweise ausgebildet sein, Druck, Temperatur und/oder Leitfähigkeit des in dem Blutkreislauf befindlichen Blutes oder des in dem Fluidkreislauf befindlichen Fluids zu messen und/oder in dem Blutkreislauf und/oder oder dem Fluidkreislauf Blut und/oder Luft zu detektieren.

Die Aktoren können beispielweise als Ventil in dem Blutkreislauf oder dem Fluidkreis oder als Pumpe in dem Blutkreislauf oder dem Fluidkreis ausgebildet sein. Auch kann ein Heizelement oder ein Heizstab einen Aktor darstellen.

Zumindest zwei der Module sind als Selbststeuerungsmodule ausgebildet. Jedes der zumindest zwei Selbststeuerungsmodule weist zumindest folgende Komponenten auf: zumindest einen der Aktoren und zumindest eine Modulsteuerungseinheit. Insbesondere können die Selbststeuerungsmodule jeweils auch zumindest einen der Sensoren aufweisen. Diese Komponenten eines jeweiligen Selbststeuerungsmoduls sind insbesondere derart miteinander verbunden, dass Daten zwischen den Komponenten ausgetauscht werden können. Die Selbststeuerungsmodule können Leitungen des Blutkreislaufs und/oder des zumindest einen Fluidkreislauf aufweisen. Es ist jedoch auch möglich, dass die offenbarungsgemäße Blutbehandlungsvorrichtung derart ausgebildet ist, dass der zumindest eine Aktor (und insbesondere gegebenenfalls auch ein Sensor) eines der Selbststeuerungsmodule auf Leitungen anderer Module der Blutbehandlungsvorrichtung Zugriff hat, so dass das entsprechende Selbststeuerungsmodul keine eigenen Leitungen aufweisen muss.

Die jeweilige Modulsteuerungseinheit ist ausgebildet, den zumindest einen Aktor desselben Selbststeuerungsmoduls, insbesondere anhand von Daten des zumindest einen Sensors desselben Selbststeuerungsmoduls, steuern zu können. Des Weiteren ist die jeweilige Modulsteuerungseinheit ausgebildet, eine vorbestimmte Zustandsänderung des zur jeweiligen Modulsteuerungseinheit gehörigen Selbststeuerungsmoduls feststellen zu können. Die vorbestimmte Zustandsänderung ist beispielsweise eine Unterbrechung oder Störung der Verbindung zwischen einer der Modulsteuerungseinheiten und dem zumindest einen Aktor desselben Selbststeuerungsmoduls oder eine Unterbrechung oder Störung der Verbindung zwischen einer der Modulsteuerungseinheiten und dem zumindest einen Sensor desselben Selbststeuerungsmoduls.

Die Modulsteuerungseinheiten sind derart ausgebildet, dass sie bei Feststellung der vorbestimmten Zustandsänderung eine entsprechende Modulzustandsänderungsmeldung direkt zu der zumindest einen anderen Modulsteuerungseinheit senden können, und entsprechend eine Modulzustandsänderungsmeldung direkt von der zumindest einen anderen Modulsteuerungseinheit empfangen zu können.

"Direkt" bedeutet, dass die zumindest zwei Modulsteuerungseinheiten ausgebildet sind, sich gegenseitig Modulzustandsänderungsmeldungen zu senden und diese entsprechend zu empfangen, ohne die Hauptsteuerungseinheit zwischenzuschalten oder beim Senden und Empfangen einer Modulzustandsänderungsmeldung miteinzubeziehen. Anders ausgedrückt, sind Modulsteuerungseinheiten ausgebildet unmittelbar miteinander zu kommunizieren.

Die zumindest zwei Modulsteuerungseinheiten sind derart ausgebildet, dass sie jeweils bei Empfang einer Modulzustandsänderungsmeldung von der zumindest einen anderen Modulsteuerungseinheit den zumindest einen Aktor desselben Selbststeuerungsmoduls, das heißt den zumindest einen Aktor des Selbststeuerungsmoduls, dessen Modulsteuerungseinheit die Modulzustandsänderungsmeldung empfangen hat, anzuweisen, eine vorbestimmte Aktion auszuführen.

Die "vorbestimmte Aktion" kann in Form eines vorbestimmten einzelnen Vorgangs oder in Form einer Abfolge mehrerer Vorgänge (in Form eines Programms oder einer Routine) umgesetzt sein.

Durch die offenbarungsgemäße Ausgestaltung wird in vorteilhafter Weise eine modulare Blutbehandlungsvorrichtung geschaffen, die schnell auf gewisse Zustandsänderungen einzelner Module reagieren kann.

Gemäß einem Aspekt der Offenbarung kann die von zumindest einer der Modulsteuerungseinheiten angewiesene vorbestimmte Aktion ein Schalten des zumindest einen Sensor und/oder des zumindest einen Aktor desselben Selbststeuerungsmoduls in eine Nullstellung und/oder ein Freischalten einer Bypassleitung desselben Selbststeuerungsmoduls für ein Fluidsystem, das heißt für den Blutkreislauf und/der den zumindest einen weiteren Fluidkreislauf, der Blutbehandlungsvorrichtung sein.

Ist der Aktor eine Pumpe, kann die Nullstellung einem ausgeschalteten Zustand der Pumpe entsprechen. Ist der Aktor ein Ventil, kann die Nullstellung einem geschlossenen Zustand des Ventils entsprechen. Die Nullstellung des zumindest einen Sensors kann einem ausgeschalteten Zustand des Sensors entsprechen.

Vorzugsweise ist die Bypassleitung eine Komponente desjenigen Selbststeuerungsmoduls, dessen Modulsteuerungseinheit die vorbestimmte Aktion anweist. Es ist jedoch auch möglich, dass die Bypassleitung eine Komponente eines anderen Moduls ist.

Ein "Freischalten einer Bypassleitung" bedeutet insbesondere, dass eine Leitung, durch welche in einem Normalbetrieb Blut und/oder ein anderes Fluid wie Dialysierflüssigkeit strömt, blockiert und das Blut und/oder das andere Fluid ausschließlich über die Bypassleitung geleitet wird. Es ist jedoch auch im Sinne der Offenbarung, die im Normalbetrieb genutzte Leitung nur teilweise zu blockieren und nur einen Teil des Blutes und/oder des anderen Fluids über die Bypassleitung zu leiten.

Wird als vorbestimmte Aktion ein Sensor und/oder Aktor in eine Nullstellung geschaltet und/oder eine Bypassleitung freigeschaltet, ist es in vorteilhafter Weise möglich, bei einer vorbestimmten Modulzustandsänderung eines Steuermoduls übrige Module schnell in einen Standbyzustand zu bringen, ohne gleich die gesamte Blutbehandlungsvorrichtung herunterfahren zu müssen.

Gemäß einem Aspekt der Offenbarung kann zumindest eines der Selbststeuerungsmodule über den zumindest einen Sensor hinaus als weitere Komponente einen Überwachungssensor aufweisen. Die Modulsteuerungseinheit des Selbststeuerungsmoduls mit dem Überwachungssensor kann eine Ausführungseinheit und eine Überwachungseinheit aufweisen. Der zumindest eine Sensor des Selbststeuerungsmoduls kann mit der Ausführungseinheit verbunden sein und der Überwachungssensor kann mit der Überwachungseinheit verbunden sein. Die Modulsteuerungseinheit kann ausgebildet sein, die vorbestimmte Modulzustandsänderung des zugehörigen Selbststeuerungsmoduls durch Abgleich von Daten der Ausführungseinheit und der Überwachungseinheit zu erkennen.

**In** einem ordnungsgemäßen Betrieb kommt der Überwachungsrechner auf der Grundlage des Überwachungssensors auf dieselben Ergebnisse beziehungsweise Daten wie der Ausführungsrechner auf der Grundlage des Sensors. Liegt eine vorbestimmte Modulzustandsänderung vor, weichen die Daten des Ausführungsrechners und des Überwachungsrechners voneinander ab, so dass die vorbestimmte Modulzustandsänderung erkannt werden kann.

Wie voranstehend geschildert, ist es möglich, eine Störung oder Unterbrechung der Verbindung zwischen einer Modulsteuerungseinheit und dem entsprechenden Aktor oder Sensor als vorbestimmte Modulzustandsänderung des Selbststeuerungsmoduls zu werten. Wird ein Überwachungssensor und eine Überwachungseinheit vorgesehen, ist es in vorteilhafter Weise möglich, das Ausmaß der Störung zu spezifizieren und einen Vorgang aus mehreren möglichen Vorgängen oder eine Abfolge mehrerer Vorgänge aus mehreren möglichen Abfolgen als die vorbestimmte Aktion entsprechend dem Ausmaß der Störung auszuwählen. Somit ist es möglich, ein Selbststeuerungsmodul angemessen auf eine Modulzustandsänderung eines anderen Selbststeuerungsmoduls reagieren zu lassen.

Gemäß einem Aspekt der Offenbarung kann ein mit einer Überwachungseinheit ausgestattetes Selbststeuerungsmodul derart ausgebildet sein, dass die Überwachungseinheit nicht nur mit dem entsprechenden Überwachungssensor, sondern auch mit dem zumindest einen Sensor verbunden ist. Somit ist es möglich, dass der Überwachungsrechnung dieselben Berechnungen wie der Ausführungsrechner zum einen auf der Grundlage des Überwachungssensors und zu anderen wie der Ausführungsrechner auf der Grundlage des zumindest einen Sensors ausführt, so dass durch Abgleich dieser beiden Berechnungen des Überwachungsrechners mit der entsprechenden Berechnung des Ausführungsrechners mittels der Modulsteuerungseinheit bzw. mittels des Überwachungsrechners der Modulsteuerungseinheit festgestellt werden kann, ob der zumindest eine Sensor oder der Ausführungsrechner ordnungsgemäß funktionieren.

Gemäß einem Aspekt der Offenbarung können sich die Bauart des zumindest einen Sensors und die Bauart des Überwachungssensors des Selbststeuerungsmoduls unterscheiden und/oder können sich die Bauart eines Mikroprozessors der Ausführungseinheit und die Bauart eines Mikroprozessors der Überwachungseinheit unterscheiden. Durch unterschiedliche Bauarten kann es ermöglicht werden, nicht nur zufällige, sondern auch systematische Ausfälle oder Beeinträchtigungen zu erkennen.

Gemäß einem Aspekt der Offenbarung kann zumindest eines der Selbststeuerungsmodule Kalibrierdaten für zumindest einen Teil der Komponenten desselben Selbststeuerungsmoduls speichern. Als Kalibierdaten kann insbesondere zumindest eine Kalibrierkurve, insbesondere Koeffizienten einer Kalibrierkurve, beispielsweise eines Polynoms, gespeichert werden. Die Speicherung von Kalibierdaten in dem Selbststeuerungsmodul kann in vorteilhafter Weise die Kalibrierung des Selbststeuerungsmoduls vereinfachen. Insbesondere kann die Kalibrierung des einzelnen Selbststeuerungsmoduls bzw. dessen Komponenten (Aktor; Sensor) unabhängig von der Blutbehandlungsvorrichtung ermöglicht beziehungsweise vereinfacht werden.

Gemäß einem Aspekt der Offenbarung kann zumindest eines der Selbststeuerungsmodule zumindest einen Schwellenwert für zumindest einen Teil der Komponenten desselben Selbststeuerungsmoduls speichern und kann die Modulsteuerungseinheit desselben Selbststeuerungsmoduls ausgebildet sein, die vorbestimmte Modulzustandsänderung des zugehörigen Selbststeuerungsmoduls durch Abgleich von Daten zumindest einer der Komponenten desselben Selbststeuerungsmoduls und dem zumindest einen Schwellenwert zu erkennen. Mittels Verwendung von Schwellenwerten kann in vorteilhafter Weise eine schnelle Erkennung der vorbestimmten Modulzustandsänderung ermöglicht werden.

Gemäß einem Aspekt der Offenbarung kann die Hauptsteuerungseinheit als eigenes Modul in Form eines Hauptsteuerungsmoduls ausgebildet sein und kann die Blutbehandlungsvorrichtung die zur Durchführung einer Blutbehandlung eines Patienten notwendigen Module mehrfach aufweisen, so dass die Blutbehandlungsvorrichtung zur gleichzeitigen Blutbehandlung mehrerer Patienten geeignet ist. Entsprechend kann das Hauptsteuerungsmodul ausgebildet sein, die gleichzeitige Blutbehandlung der mehreren Patienten steuern zu können. Das Vorsehen eines Hauptsteuerungsmoduls kann in vorteilhafter Weise die gleichzeitige Behandlung mehrerer Patienten vereinfachen und die Kosten einer gleichzeitigen Behandlung mehrerer Patienten senken.

Gemäß einem Aspekt der Offenbarung kann die Blutbehandlungsvorrichtung ein Bedien- und Anzeigemodul aufweisen, das ausgebildet ist, Daten zumindest einzelner Module der Behandlungsvorrichtung anzuzeigen, Befehle von einem Benutzer zu empfangen und die Befehle an die Hauptsteuerungseinheit und/oder direkt an zumindest einzelne Module der Behandlungsvorrichtung weiterzuleiten. Insbesondere kann das Bedien- und Anzeigemodul ausgebildet sein, selbst die Hauptsteuerungseinheit aufzuweisen. Durch Vorsehen eines separaten Bedien- und Anzeigemoduls ist es in einfacher Weise möglich, die Mensch-Maschine-Schnittstelle gegebenenfalls an neue Standards anzupassen, ohne die gesamte Blutbehandlungsvorrichtung überarbeiten zu müssen. Insbesondere kann ein Bedien- und Anzeigemodul derart ausgebildet sein, dass es, insbesondere gleichzeitig, für mehrere Behandlungsvorrichtungen verwendet werden kann.

Gemäß einem Aspekt der Offenbarung kann die Blutbehandlungsvorrichtung ein Kalibriermodul aufweisen, welches zumindest ein Referenzmessgerät aufweist, das ausgebildet ist, einen Abgleich mit zumindest einem der Sensoren zu ermöglichen. Mittels eines Kalibriermoduls kann in vorteilhafter Weise eine Kalibrierung einzelner Module vereinfacht werden. Insbesondere kann eine solche Kalibrierung in einem Zustand vorgenommen werden, in welchem das zu kalibrierende Modul mit keinem anderen oder nicht mit allem zu einer Blutbehandlung notwendigen Module verbunden ist (zum Beispiel vor dem Zusammenbau, bei einem Austausch eines Moduls). Mittels des Kalibriermoduls kann der Instandhaltungsaufwand (Instandhaltungszeit und/oder -kosten) der offenbarungsgemäßen Blutbehandlungsvorrichtung geringgehalten werden. Das Kalibriermodul ist insbesondere derart ausgebildet, dass es während einer Behandlung nicht Bestandteil der Blutbehandlungsvorrichtung ist.

### Kurzbeschreibung der Zeichnungen

Die offenbarungsgemäße Blutbehandlungsvorrichtung wird im Folgenden anhand bevorzugter Ausführungsbeispiele unter Bezugnahme auf die beigefügten Zeichnungen näher beschrieben. Es zeigen:
Fig. 1 eine schematische Ansicht einer offenbarungsgemäßen Blutbehandlungsvorrichtung, einer Stromversorgung, einer Wasserver- und entsorgung, einer zentralen Konzentratversorgung und eines Patienten;
Fig. 2 eine schematische Detailansicht eines offenbarungsgemäßen Selbststeuerungsmoduls in Form eines Wasseraufbereitungsmoduls;
Fig. 3 eine schematische Detailansicht eines offenbarungsgemäßen Selbststeuerungsmoduls in Form eines Dialysierflüssigkeitsaufbereitungsmoduls;
Fig. 4 eine schematische Detailansicht eines Bilanzierungsmoduls;
Fig. 5 eine schematische Detailansicht eines offenbarungsgemäßen Selbststeuerungsmoduls in Form eines Dialysierflüssigkeitsanschlussmoduls;
Fig. 6 eine schematische Detailansicht eines offenbarungsgemäßen Selbststeuerungsmoduls in Form eines Hämodiafiltrationsmoduls und
Fig. 7 eine schematische Detailansicht eines offenbarungsgemäßen Selbststeuerungsmoduls in Form eines Blutanschlussmoduls und eines Dialysators.

### Detaillierte Beschreibung bevorzugter Ausführungsformen

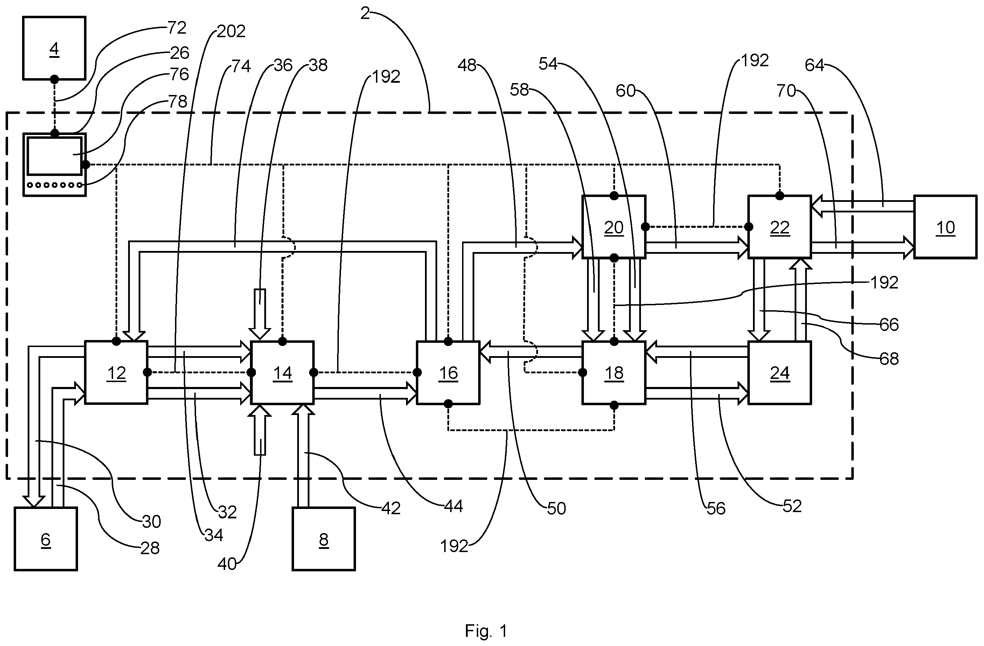

Fig. 1 zeigt eine schematische Ansicht einer offenbarungsgemäßen Blutbehandlungsvorrichtung 2, einer Stromversorgung 4, einer Wasserver- und entsorgung 6, einer zentralen Konzentratversorgung 8 und eines Patienten 10.

Die Blutbehandlungsvorrichtung 2 weist ein Wasseraufbereitungsmodul 12, ein Dialysierflüssigkeitsaufbereitungsmodul 14, ein Bilanzierungsmodul 16, ein Dialysierflüssigkeitsanschlussmodul 18, ein Hämodiafiltrationsmodul 20, ein Blutanschlussmodul 22, einen Dialysator 24 und ein Hauptsteuerungsmodul 26 auf.

Das Wasseraufbereitungsmodul 12 weist (nicht näher gezeigte) Schnittstellen (Anschlüsse oder Ports) auf, an welche eine Frischwasserleitung und eine Abwasserleitung angeschlossen werden können, um mit der Wasserver- und entsorgung 6 verbunden zu werden, so dass das Wasseraufbereitungsmodul 12 einen Frischwasserstrom 28 beziehen und einen Abwasserstrom 30 abführen kann. Des Weiteren weist das Wasseraufbereitungsmodul 12 (nicht näher gezeigte) Schnittstellen auf, über welche aufbereitetes Wasser in einem Hauptstrom 32 und in einem Nebenstrom 34 an das Dialysierflüssigkeitsaufbereitungsmodul 14 abgegeben werden kann. Über eine (nicht näher gezeigte) Schnittstelle kann das Wasseraufbereitungsmodul 12 einen Dialysatstrom 36 von dem Bilanzierungsmodul 16 aufnehmen.

Das Dialysierflüssigkeitsaufbereitungsmodul 14 weist (nicht näher gezeigte) Schnittstellen auf, über die der Hauptstrom 32 und der Nebenstrom 34 des von dem Wasseraufbereitungsmodul 12 aufbereiteten Wassers aufgenommen werden können. Des Weiteren weist das Dialysierflüssigkeitsaufbereitungsmodul 14 (nicht näher gezeigte) Schnittstellen auf, über die das Dialysierflüssigkeitsaufbereitungsmodul 14 einen basischen Konzentratstrom 38 und einen sauren Konzentratstrom 40 aus entsprechenden (nicht gezeigten) Kanistern oder Kartuschen aufnehmen kann. Über zumindest eine (nicht näher gezeigte) Schnittstelle kann das Dialysierflüssigkeitsaufbereitungsmodul 14 einen sauren Konzentratstrom 42 aus der zentralen Konzentratversorgung 8 aufnehmen. Über eine (nicht näher gezeigte) Schnittstelle kann das Dialysierflüssigkeitsaufbereitungsmodul 14 einen Dialysierflüssigkeitsstrom 44 an das Bilanzierungsmodul 16 abgeben.

Das Bilanzierungsmodul 16 weist eine (nicht näher gezeigte) Schnittstelle auf, über die der Dialysierflüssigkeitsstrom 44 von dem Dialysierflüssigkeitsaufbereitungsmodul 14 aufgenommen werden kann. Über eine (nicht näher gezeigte) Schnittstelle kann das Bilanzierungsmodul 16 darüber hinaus einen Dialysierflüssigkeitsstrom 48 an das Hämodiafiltrationsmodul 20 abgeben. Des Weiteren weist das Bilanzierungsmodul 16 (nicht näher gezeigte) Schnittstellen auf, über die ein Dialysatstrom 50 von dem Dialysierflüssigkeitsanschlussmodul 18 aufgenommen werden kann und der Dialysatstrom 36 an das Wasseraufbereitungsmodul 12 abgegeben werden kann.

Das Dialysierflüssigkeitsanschlussmodul 18 weist eine (nicht näher gezeigte) Schnittstelle auf, über die ein Dialysierflüssigkeitsstrom 52 an den Dialysator 24 abgegeben werden kann. Des Weiteren weist das Dialysierflüssigkeitsanschlussmodul 18 eine (nicht näher gezeigte) Schnittstelle auf, über die ein Dialysierflüssigkeitsstrom 54 von dem Hämodiafiltrationsmodul 20 aufgenommen werden kann. Über (nicht näher gezeigte) Schnittstellen kann das Dialysierflüssigkeitsanschlussmodul 18 einen Dialysatstrom 56 von dem Dialysator 24 aufnehmen und den Dialysatstrom 50 an das Bilanzierungsmodul 16 abgeben. Des Weiteren weist das Dialysierflüssigkeitsanschlussmodul 18 eine (nicht näher gezeigte) Schnittstelle auf, über die ein Strom 58 überschüssigen Substituats von dem Hämodiafiltrationsmodul 20 aufgenommen werden kann.

Das Hämodiafiltrationsmodul 20 weist (nicht näher gezeigte) Schnittstellen auf, über die der Dialysierflüssigkeitsstrom 48 von dem Bilanzierungsmodul 16 aufgenommen werden kann, der Dialysierflüssigkeitsstrom 54 an das Dialysierflüssigkeitsanschlussmodul 18 abgegeben werden kann und ein Substituatstrom 60 an das Blutanschlussmodul 22 abgegeben werden kann. Des Weiteren weist das Hämodiafiltrationsmodul 20 eine (nicht näher gezeigte) Schnittstelle auf, über die der Strom 58 überschüssigen Substituats an das Dialysierflüssigkeitsanschlussmodul 18 abgegeben werden kann.

Das Blutanschlussmodul 22 weist (nicht näher gezeigte) Schnittstellen auf, über die ein Strom 64 unbehandelten Bluts (arterielles Blut) von dem Patienten 10 aufgenommen werden kann und ein Strom 66 unbehandelten Bluts an den Dialysator 24 abgegeben werden kann. Über (nicht näher gezeigte) Schnittstellen kann das Blutanschlussmodul 22 einen Strom 68 behandelten Bluts von dem Dialysators 24 aufnehmen und einen Strom 70 behandelten Bluts (venöses Blut) an den Patienten 10 abgeben. Des Weiteren weist das Blutanschlussmodul 22 eine (nicht näher gezeigte) Schnittstelle auf, über die der Substituatstrom 60 von dem Hämodiafiltrationsmodul 20 aufgenommen werden kann.

Der Dialysator 24 weist Schnittstellen auf, über die der Dialysierflüssigkeitsstrom 52 von dem Dialysierflüssigkeitsanschlussmodul 18 aufgenommen werden kann und der Dialysatstrom 56 an das Dialysierflüssigkeitsanschlussmodul 18 abgegeben werden kann. Des Weiteren weist der Dialysator 24 Schnittstellen auf, über die der Strom 66 unbehandelten Bluts von dem Blutanschlussmodul 22 aufgenommen werden kann und der Strom 68 behandelten Bluts an das Blutanschlussmodul 22 abgegeben werden kann.

Das Hauptsteuerungsmodul 26 kann mit der Stromversorgung 4 über eine elektrische Leitung 72 verbunden werden. Die Blutbehandlungsvorrichtung 2 weist Leitungen 74 auf, mittels derer das Hauptsteuerungsmodul 26 die Module 12 bis 22 mit elektrischem Strom beziehungsweise elektrischer Leistung versorgen kann. Die Leitungen 74 sind darüber hinaus ausgebildet, Daten (Befehle von dem Hauptsteuerungsmodul 26 an eines der Module 12 bis 22, Rückmeldungen von einem der Module 12 bis 22 an das Hauptsteuerungsmodul 26) zwischen dem Hauptsteuerungsmodul 26 und den Modulen 12 bis 22 auszutauschen zu können. Das Hauptsteuerungsmodul 26 ist auch als offenbarungsgemäßes Bedien- und Anzeigemodul ausgebildet und weist einen berührungsempfindlichen Bildschirm 76 und Eingabetasten 78 auf. Das Hauptsteuerungsmodul 26 kann alternativ auch ohne integrierten Bildschirm und/oder ohne integrierte Eingabetasten ausgebildet sein. Insbesondere kann das Hauptsteuerungsmodul 26 derart ausgebildet, dass ein separater Bildschrim und/oder eine separate Tastatur angeschlossen werden kann. Insbesondere können der Bildschirm 76 und/oder die Eingabetasten 78 in einem separat vom Hauptsteuerungsmodul 26 ausgebildeten Bedien- und Anzeigemodul ausgebildet sein.

**Im** Folgenden werden die einzelnen Module 12 bis 22 in einem Zustand während einer Blutbehandlung beschrieben, in welchem die Module 12 bis 26 miteinander verbunden sind, um die Blutbehandlungsvorrichtung 2 zu bilden, und die Blutbehandlungsvorrichtung 2 mit der Stromversorgung 4, der Wasserver- und entsorgung 6, der zentralen Konzentratversorgung 8 und dem Patienten 10 verbunden ist.

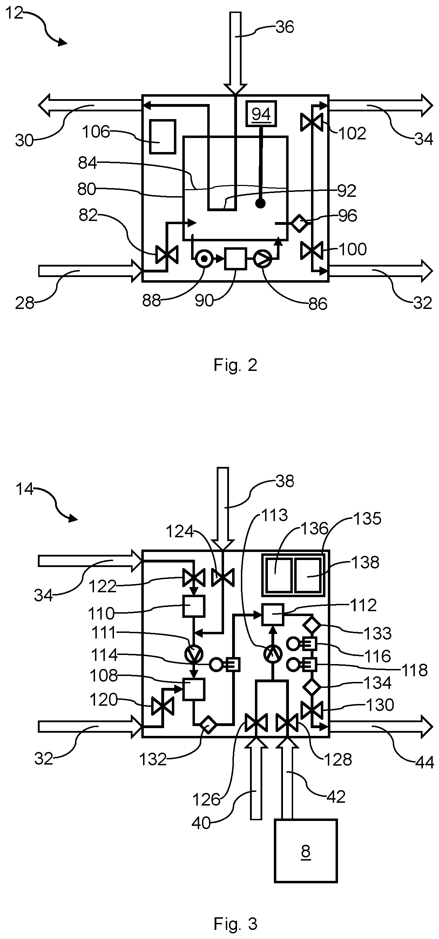

Fig. 2 zeigt eine schematische Detailansicht des Wasseraufbereitungsmoduls 12. Für eine Blutbehandlung muss Wasser zunächst entgast und temperiert werden. Das Wasseraufbereitungsmoduls 12 umfasst einen Vorlaufbehälter 80 (Wassertank). Über ein Ventil 82 und ein (nicht gezeigtes) Druckminderventil kann der Frischwasserstrom 28 von der Wasserver- und entsorgung 6 in den Vorlaufbehälter 80 geleitet werden. Zwei (nicht gezeigte) Niveausensoren bestimmen dabei den Füllstand 84. Zur Entgasung wird eine Pumpe 86 eingesetzt, die das Wasser im Vorlaufbehälter 80 zirkulieren lässt und dabei einen mithilfe eines Drucksensors 88 überwachten Unterdruck erzeugt, wodurch gelöste Gase mittels einer Entgasungskammer 90 entweichen können. Im Vorlaufbehälter 80 befindet sich außerdem ein Wärmetauscher 92 zur Energierückgewinnung aus dem Dialysatstrom 36. Das warme Dialysat fließt vom Dialysator 24 kommend über das Dialysierflüssigkeitsmodul 18 und das Bilanzierungsmodul 16 in den Wärmetauscher 92, wo es Wärme abgibt, und anschließend als Abwasserstrom in den Abfluss. Zur Erwärmung des Wassers kommen außerdem ein in dem Vorlaufbehälter 80 befindliches Heizelement 94 sowie ein Temperatursensor 96 zum Einsatz. Mit dem Temperatursensor 96 kann die Temperatur des aufbereiteten Wassers gemessen werden, welches nach dem Temperatursensor 96 zum einen dem Hauptstrom 32 und zum anderen dem Nebenstrom 34 zugeleitet wird. Über Ventile 100 und 102 können der Hauptstrom 32 aufbereiteten Wassers und der Nebenstrom 34 aufbereiteten Wassers unterbunden werden.

Die Ventile 82, 100 und 102, die Pumpe 86 und das Heizelement 94 stellen offenbarungsgemäße Aktoren dar. Der Drucksensor 88 sowie der Temperatursensor 96 stellen offenbarungsgemäße Sensoren dar. Das Wasseraufbereitungsmodul 12 weist eine Modulsteuerungseinheit 106 auf, die über (nicht gezeigte) Leitungen mit den Ventilen 82, 100 und 102, der Pumpe 86, dem Drucksensor 88, dem Heizelement 94 und dem Temperatursensor 96 verbunden ist. Des Weiteren ist die Modulsteuerungseinheit 106 über eine der Leitungen 74 mit dem Hauptsteuerungsmodul 26 verbunden. Das Wasseraufbereitungsmodul 12 ist als offenbarungsgemäßes Selbststeuerungsmodul ausgebildet. Die Modulsteuerungseinheit 106 steuert bzw. regelt die Pumpe 86 auf der Grundlage des vom Drucksensor 88 gemessenen Unterdrucks, um einen vorbestimmten Soll-Unterdruck zu erreichen. Außerdem steuert bzw. regelt die Modulsteuerungseinheit 106 das Heizelement 94 auf der Grundlage der von dem Temperatursensor 96 gemessenen Temperaturen, um eine vorbestimmte Soll-Temperatur zu erreichen.

Bei der Durchführung einer Blutbehandlung gibt das Hauptsteuerungsmodul 26 der Modulsteuerungseinheit 106 des Wasseraufbereitungsmoduls 12 den vorbestimmten Soll-Unterdruck und die Soll-Temperatur vor. Auf die Pumpe 86 und das Heizelement 94 hat das Hauptsteuerungsmodul 26 keinen unmittelbaren Zugriff und erteilt zum Beispiel keine Anweisungen bezüglich einer Drehzahl der Pumpe 86 oder einer Wärmeleistung des Heizelements 94.

Fig. 3 zeigt eine schematische Detailansicht des Dialysierflüssigkeitsaufbereitungsmoduls 14. Zur Aufbereitung von Dialysierflüssigkeit wird dem Hauptstrom 32 des aufbereiteten Wassers in einer Mischkammer 108 zunächst eine basische Komponente (zum Beispiel Natriumhydrogencarbonat) zugegeben. Die basische Komponente kann zu einem von einem (nicht gezeigten) Konzentratkanister bezogen werden (siehe basischen Konzentratstrom 38), der die basische Komponente in einem bereits gelösten Zustand enthält (zum Beispiel gelöstes Natriumhydrogencarbonat). Zum anderen kann die basische Komponente von einer Kartusche 110 bezogen werden, welche die basische Komponente in einem pulverförmigen Zustand enthält (zum Beispiel pulverförmiges Natriumhydrogencarbonat). Um eine Vermischung der pulverförmigen basischen Komponente mit dem Hauptstrom 32 des aufbereiteten Wassers zu erleichtern, wird zunächst der Nebenstrom 34 des aufbereiteten Wassers mit der pulverförmigen basischen Komponente vermischt, bevor das Gemisch aus dem Nebenstroms 34 des aufbereiteten Wassers und der basischen Komponente in der Mischkammer 108 mit dem Hauptstrom 32 des aufbereiteten Wassers vermischt wird. Unabhängig davon, ob die basische Komponente von der Kartusche 110 oder von dem (nicht gezeigten) Konzentratkanister bezogen wird, wird die basische Komponente über eine stromaufwärts der Mischkammer 108 angeordneten Pumpe 111 zu der Mischkammer 108 gefördert. Das mit der basischen Komponente vermischte Wasser wird anschließend in eine Mischkammer 112 geleitet, in welcher eine saure Komponente zugegeben wird. Die saure Komponente kann aus unterschiedlichen Quellen bezogen werden. Zum einen kann die saure Komponente von einem (nicht gezeigten) Konzentratkanister bezogen werden und zum anderen kann die zentrale Konzentratversorgung 8 das Dialysierflüssigkeitsaufbereitungsmodul 14 mit der sauren Komponente versorgen. Zum Anschluss an die die zentrale Konzentratversorgung 8 können mehrere Leitungen vorgesehen werden, um bei Bedarf unterschiedlich zusammengesetzte Konzentrate beziehen zu können. Unabhängig von der Bezugsquelle wird die saure Komponente über eine stromaufwärts der Mischkammer 112 angeordneten Pumpe 113 zu der Mischkammer 112 gefördert.

Zur Bestimmung der Zusammensetzung der Dialysierflüssigkeit kommen die Leitfähigkeitssensoren 114 und 116 zum Einsatz. Der Leitfähigkeitssensor 114 misst die Leitfähigkeit nach Zugabe der basischen Komponente (Natriumhydrogencarbonat) zum entgasten und erwärmten Wasser. Der Leitfähigkeitssensor 116 bestimmt die Gesamtleitfähigkeit nach Zugabe der sauren Komponente. Da eine falsch zusammengesetzt Dialysierflüssigkeit unter Umständen dem Patienten 10 schaden kann, weist das Dialysierflüssigkeitsaufbereitungsmodul 14 einen weiteren Leitfähigkeitssensor 118 auf, der ebenfalls die Gesamtleitfähigkeit bestimmt. Der Leitfähigkeitssensor 118 stellt einen offenbarungsgemäßen Überwachungssensor bezüglich des Leitfähigkeitssensors 116 dar.

Das Dialysierflüssigkeitsaufbereitungsmodul 14 weist ein Ventil 120 zur Steuerung des Zuflusses des Hauptstroms 32 des aufbereiteten Wassers, ein Ventil 122 zur Steuerung des Zuflusses des Nebenstroms 34 des aufbereiteten Wassers, ein Ventil 124 zur Steuerung des Zuflusses des basischen Kozentratstroms 38, ein Ventil 126 zur Steuerung des Zuflusses des sauren Konzentratstroms 40, ein Ventil 128 zur Steuerung des Zuflusses des sauren Konzentratstroms 42 aus der zentralen Konzentratversorgung 8 und ein Ventil 130 zur Steuerung des Abflusses des Dialysierflüssigkeitsstroms 44 auf. Dem Leitfähigkeitssensoren 114 ist ein Temperatursensor 132 vorgeschaltet. Den Leitfähigkeitssensoren 116 und 118 ist ein Temperatursensor 133 vorgeschaltet und ein Temperatursensor 134 nachgeschaltet. Der Temperatursensor 134 stellt einen offenbarungsgemäßen Überwachungssensor bezüglich des Temperatursensors 133 dar.

Anstelle des in dem Wasseraufbereitungsmodul 12 ausgebildeten Ventil 100 und dem dazu redundant in dem Dialysierflüssigkeitsaufbereitungsmodul 14 ausgebildeten Ventil 120 im Hauptstrom 32 des aufbereiteten Wassers ist alternativ möglich, nur ein entsprechendes Ventil in einem der beiden Module 12 oder 14 vorzusehen. Anstelle des in dem Wasseraufbereitungsmodul 12 ausgebildeten Ventil 102 und dem dazu redundant in dem Dialysierflüssigkeitsaufbereitungsmodul 14 ausgebildeten Ventil 122 im Nebenstrom 34 des aufbereiteten Wassers ist alternativ möglich, nur ein entsprechendes Ventil in einem der beiden Module 12 oder 14 vorzusehen.

Die Pumpen 111 und 113 sowie die Ventile 120 bis 130 stellen offenbarungsgemäße Aktoren dar. Die Leitfähigkeitssensoren 114 bis 118 sowie die Temperatursensoren 132, 133 und 134 stellen offenbarungsgemäße Sensoren dar. Das Dialysierflüssigkeitsaufbereitungsmodul 14 weist eine Modulsteuerungseinheit 135 auf, die über (nicht gezeigte) Leitungen mit den Pumpen 111 und 113, den Ventilen 120 bis 130, den Leitfähigkeitssensoren 114 bis 118 sowie den Temperatursensoren 132, 133 und 134 verbunden ist. Des Weiteren ist die Modulsteuerungseinheit 135 über eine der Leitungen 74 mit dem Hauptsteuerungsmodul 26 verbunden. Das Dialysierflüssigkeitsaufbereitungsmodul 14 ist als offenbarungsgemäßes Selbststeuerungsmodul ausgebildet. Die Modulsteuerungseinheit 135 weist eine Ausführungseinheit 136 auf, welche die Pumpe 111 und die Ventile 120 bis 124 auf der Grundlage der von dem Leitfähigkeitssensor 114 gemessenen Leitfähigkeit steuert bzw. regelt, um eine vorbestimmte Soll-Leitfähigkeit des basischen Gemischs zu erreichen. Die Ausführungseinheit 136 der Modulsteuerungseinheit 135 steuert bzw. regelt die Pumpe 113 und die Ventile 126 und 128 auf der Grundlage der von dem Leitfähigkeitssensor 116 gemessenen Leitfähigkeit, um eine vorbestimmte Soll-Leitfähigkeit der Dialysierflüssigkeit zu erreichen.

Bei der Durchführung einer Blutbehandlung gibt das Hauptsteuerungsmodul 26 der Modulsteuerungseinheit 135 des Dialysierflüssigkeitsaufbereitungsmodul 14 die vorbestimmte Soll-Leitfähigkeit des basischen Gemischs und die Soll-Leitfähigkeit der Dialysierflüssigkeit vor. Optional kann die Blutbehandlungsvorrichtung 2 auch derart ausgebildet sein, dass das Hauptsteuerungsmodul dem Dialysierflüssigkeitsaufbereitungsmodul 14 Soll-Konzentrationen (zum Beispiel von Hydrogencarbonat und Natrium) vorgibt und die Modulsteuerungseinheit 135 des Dialysierflüssigkeitsaufbereitungsmoduls 14 die Soll-Konzentrationen dann in die Soll-Leitfähigkeiten umrechnet.

Die Modulsteuerungseinheit 135 des Dialysierflüssigkeitsaufbereitungsmoduls 14 weist des Weiteren eine Überwachungseinheit 138 auf, welche über (nicht gezeigte) Leitungen wie die Ausführungseinheit 136 mit den Pumpen 111 und 113, den Ventilen 120 bis 130, den Leitfähigkeitssensoren 114 bis 118 sowie den Temperatursensoren 132 bis 134 verbunden ist und welche bei der Durchführung einer Blutbehandlung dieselben Berechnungsprozesse wie die Ausführungseinheit 136 durchführt. Die Überwachungseinheit 138 ist über eine (nicht gezeigte) Leitung mit der Ausführungseinheit 136 verbunden. Außerdem ist die Überwachungseinheit 138 ausgebildet, Ergebnisse ihrer eigenen Berechnungen mit entsprechenden Ergebnissen der Ausführungseinheit 136 abzugleichen, so dass die Modulsteuerungseinheit 135 registrieren kann, ob die Berechnungen der Ausführungseinheit 136 und der Überwachungseinheit 138 übereinstimmen.

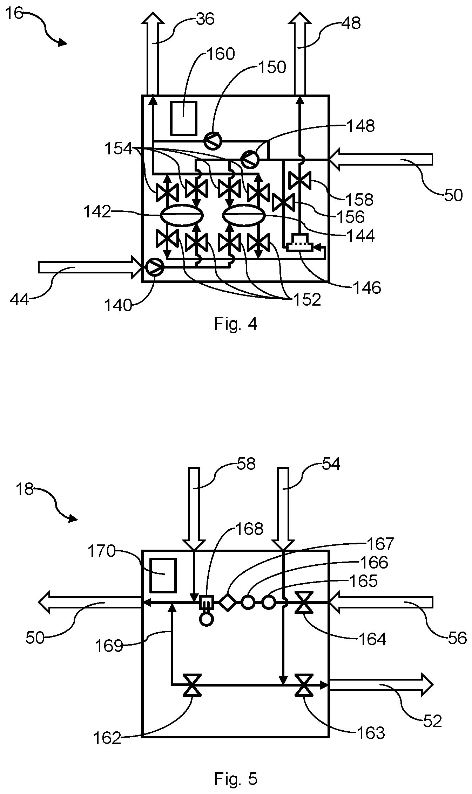

Fig. 4 zeigt eine schematische Detailansicht des Bilanzierungsmoduls 16. In dem Bilanzierungsmodul 16 wird der von dem Dialysierflüssigkeitsaufbereitungsmodul 14 kommende Dialysierflüssigkeitsstrom 44 mittels einer Dialysierflüssigkeitspumpe 140 über dialysierflüssigkeitsseitige Teile zweier Bilanzkammern 142 und 144 zu einem Dialysierflüssigkeitsfilter 146 geleitet, von welchem aus die gefilterte Dialysierflüssigkeit dem Dialysierflüssigkeitsstrom 48 zu dem Hämodiafiltrationsmodul 20 zugeleitet wird. Das Dialysat des von dem Dialysierflüssigkeitsanschlussmodul 18 kommenden Dialysatstrom 50 wird von einer Dialysatpumpe 148 über dialysatseitige Teile der Bilanzkammern 142 und 144 zu dem Dialysatstrom 36 gefördert. Um dem Patienten 10 eine definierte Menge Flüssigkeit zu entziehen, kann ein Teil des Dialysats des von dem Dialysierflüssigkeitsanschlussmodul 18 kommenden Dialysatstroms 50 über eine Ultrafiltratpumpe 150 an den dialysatseitigen Teilen der Bilanzkammern 142 und 144 vorbei direkt zu dem Dialysatstrom 36 gefördert werden.

Der Dialysierflüssigkeitsfilter 146 ist derart über ein Ventil 156 mit dem Dialysatstrom 36 verbunden, dass der von dem Dialysierflüssigkeitsfilter 146 zurückgehaltene Teil der Dialysierflüssigkeit des Dialysierflüssigkeitsstrom 44 dem Dialysatstrom 36 durch Öffnen des Ventils 156 zugeleitet werden kann. Der von dem Dialysierflüssigkeitsfilter 146 zurückgehaltene Teil der Dialysierflüssigkeit des Dialysierflüssigkeitsstrom 44 wird bei geöffnetem Ventil 156 stromaufwärts der zueinander parallelgeschalteten Pumpen 148 und 150 in den Dialysatstrom 36 eingeleitet. Bei einem Normalbetrieb ist das Ventil 156 geschlossen.

Zum Funktionieren der Bilanzkammern 142 und 144 weist das Bilanzierungsmodul 16 Ventile 152 und 154 auf, die in bekannter Weise geschaltet werden (siehe zum Beispiel DE 10 2017 125 962 A1). Des Weiteren weist das Bilanzierungsmodul 16 ein Ventil 158 auf, mittels dessen der Zufluss zu dem Dialysierflüssigkeitsstrom 48 gesteuert werden kann. Zur Überwachung bzw. Erkennung einer Membranstellung ist je Bilanzkammer 142 bzw. 144 ein (nicht gezeigter) Sensor vorgesehen.

Die Dialysierflüssigkeitspumpe 140, die Ventile 152, die Dialysatpumpe 148, die Ultrafiltratpumpe 150 sowie die Ventile 154 und 158 stellen offenbarungsgemäße Aktoren dar. Das Bilanzierungsmodul 16 weist eine Modulsteuerungseinheit 160 auf, die über (nicht gezeigte) Leitungen mit der Dialysierflüssigkeitspumpe 140, den Ventilen 152, der Dialysatpumpe 148, der Ultrafiltratpumpe 150 sowie den Ventilen 154 und 158 verbunden ist. Des Weiteren ist die Modulsteuerungseinheit 160 über eine der Leitungen 74 mit dem Hauptsteuerungsmodul 26 verbunden. Das Bilanzierungsmodul 16 ist als offenbarungsgemäßes Selbststeuerungsmodul ausgebildet. Die Modulsteuerungseinheit 160 steuert die Dialysierflüssigkeitspumpe 140, die Ventile 152, die Dialysatpumpe 148, die Ultrafiltratpumpe 150 sowie die Ventile 154 und 158 entsprechend einer von dem Hauptsteuerungsmodul 26 vorgegebenen Flussrate der Dialysierflüssigkeit und entsprechend einem Ultrafiltrationsvolumen.

Fig. 5 zeigt eine schematische Detailansicht des Dialysierflüssigkeitsanschlussmoduls 18. In dem Dialysierflüssigkeitsanschlussmodul 18 wird der vom Dialysator 24 kommende Dialysatstrom 56 zu dem zum Bilanzierungsmodul 16 führenden Dialysatstrom 50 geleitet. Der vom Hämodiafiltrationsmodul 20 kommende Dialysierflüssigkeitsstrom 54 kann in dem Dialysierflüssigkeitsanschlussmodul 18 in den zum Dialysator 24 führenden Dialysierflüssigkeitsstrom 52 und /oder über eine Bypassleitung 169 in den zum Bilanziergungsmodul 16 führenden Dialysatstrom 50 eingespeist werden. Der von dem Hämodiafiltrationsmodul 20 kommende Strom 58 überschüssigen Substituats kann in dem Dialysierflüssigkeitsanschlussmodul 18 in den zum Bilanzierungsmodul 16 führenden Dialysatstrom 50 eingespeist werden. Das Dialysierflüssigkeitsanschlussmodul 18 weist Ventile 162, 163 und 164 auf. Mittels des Ventils 162 kann der Zufluss vom Dialysierflüssigkeitsstrom 54 zum Dialysatstrom 50 bzw. die Durchströmung der Bypassleitung 169 gesteuert werden. Mittels des Ventils 163 kann der Zufluss vom Dialysierflüssigkeitsstrom 54 zum Dialysierflüssigkeitsstrom 52 gesteuert werden. Mittels des Ventils 164 kann der Zufluss vom Dialysatstrom 56 zum Dialysatstrom 50 gesteuert werden. Wird das Ventil 163 vollständig geschlossen und wird gleichzeitig das Ventil 162, insbesondere vollständig, geöffnet, kann der Dialysierflüssigkeitsstrom 54 über die Bypassleitung 169 vollständig in den Dialysatstrom 50 eingespeist werden. Stromabwärts des Ventils 164 sind ein Blutleckdetektor 165 sowie optional ein UV-Sensor 166, ein Temperatursensor 167 und/oder ein Leitfähigkeitssensor 168 angeordnet.

Die Ventile 162 bis 164 stellen offenbarungsgemäße Aktoren dar. Der Blutleckdetektor 165, der optionale UV-Sensor 166, der optionale Temperatursensor 167 und/oder der optionale Leitfähigkeitssensor 168 stellen offenbarungsgemäße Sensoren dar. Das Dialysierflüssigkeitsanschlussmodul 18 weist eine Modulsteuerungseinheit 170 auf, die über (nicht gezeigte) Leitungen mit den Ventilen 162 bis 164, dem Blutleckdetektor 165 und gegebenenfalls mit dem UV-Sensor 166, dem Temperatursensor 167 und/oder dem Leitfähigkeitssensor 168 verbunden ist. Des Weiteren ist die Modulsteuerungseinheit 170 über eine der Leitungen 74 mit dem Hauptsteuerungsmodul 26 verbunden. Das Dialysierflüssigkeitsanschlussmodul 18 ist als offenbarungsgemäßes Selbststeuerungsmodul ausgebildet. Das Dialysierflüssigkeitsanschlussmodul 18 kann die Ventile 162 bis 164 entsprechend einer Vorgabe von dem Hauptsteuerungsmodul 26 ansteuern und beispielsweise auf Anweisung die Bypassleitung 169 aktivieren und die Zufuhr von Dialyseflüssigkeit zum Dialysator 24 unterbinden. Auch ist die Modulsteuerungseinheit 170 ausgebildet, die Bypassleitung 169 zu aktivieren und die Zufuhr von Dialyseflüssigkeit zum Dialysator 24 zu unterbinden, wenn der Blutleckdetektor 165, der optionale UV-Sensor 166, der optionale Temperatursensor 167 und/oder der optionale Leitfähigkeitssensor 168 jeweils oder in Zusammenschau einen oder eine vorbestimmte Menge an Werten messen bzw. misst, welcher bzw. welche eine Fehlfunktion des Dialysators 24 und/oder eine Komplikation bei dem Patienten 10 impliziert bzw. implizieren. Die Modulsteuerungseinheit 170 detektiert die Fehlfunktion des Dialysators 24 und/oder die Komplikation bei dem Patienten 10, indem sie den gemessenen Wert und/oder die vorbestimmte Menge an gemessenen Werten mit einem oder mehreren entsprechenden in der Modulsteuerungseinheit 170 oder in dem Hauptsteuerungsmodul 26 hinterlegten Referenzwerten vergleicht und eine vorbestimmte Abweichung von dem oder den Referenzwerten feststellt.

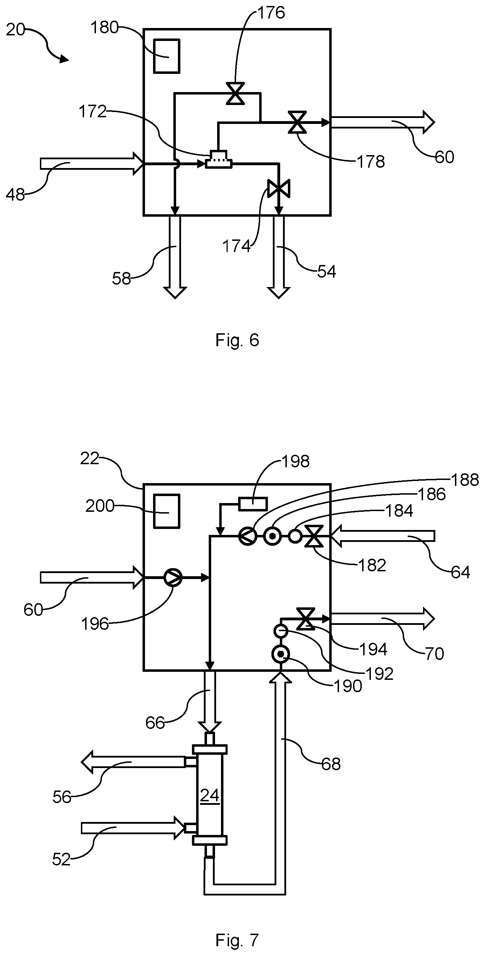

Fig. 6 zeigt eine schematische Detailansicht des Hämodiafiltrationsmoduls 20. In dem Hämodiafiltrationsmodul 20 kann die Dialysierflüssigkeit des Dialysierflüssigkeitsstroms 48 zu einem Hämodiafiltrationsfilter 172 geleitet werden, von welchem aus die gefilterte Dialysierflüssigkeit als Substituat zum einen über ein Ventil 178 zu dem Substituatstrom 60 und zum anderen über ein Ventil 176 zu dem Strom 58 überschüssigen Substituats geführt werden kann. Der von dem Hämodiafiltrationsfilter 172 zurückgehaltene Teil der von dem Dialysierflüssigkeitsstrom 48 kommenden Dialysierflüssigkeit kann über ein Ventil 174 zu dem Dialysierflüssigkeitsstrom 54 geleitet werden.

Anstelle der zueinander redundant ausgebildeten Ventile 164 des Dialysierflüssigkeitsanschlussmoduls 18 und 174 des Hämodiafiltrationsmoduls 20 ist es alternativ möglich, nur eines der beiden Ventile 164 und 174 vorzusehen.

Die Ventile 174 bis 178 stellen offenbarungsgemäße Aktoren dar. Das Hämodiafiltrationsmodul 20 weist eine Modulsteuerungseinheit 180 auf, die über (nicht gezeigte) Leitungen mit den Ventilen 174 bis 178 verbunden ist. Des Weiteren ist die Modulsteuerungseinheit 180 über eine der Leitungen 74 mit dem Hauptsteuerungsmodul 26 verbunden. Das Hämodiafiltrationsmodul 20 ist als offenbarungsgemäßes Selbststeuerungsmodul ausgebildet. Das Hämodiafiltrationsmodul 20 kann die Ventile 174 bis 178 entsprechend einer Vorgabe von dem Hauptsteuerungsmodul 26 ansteuern und beispielsweise auf Anweisung die Zufuhr von Substituat zum Patienten 10 unterbinden. Um die Einleitung des Substituats in das Blut zu unterbinden, kann das Ventil 178 geschlossen und das Ventil 176 geöffnet werden, so dass das Substituat in den Strom 58 überschüssigen Substituats eingespeist wird und zum Dialysierflüssigkeitsanschlussmodul 18 fließt, wo es in den Dialysatstrom 50 eingespeist wird.

Fig. 7 zeigt eine schematische Detailansicht des Blutanschlussmoduls 22 und des Dialysators 24. Das Blutanschlussmodul 22 ist ausgebildet, über (nicht gezeigte) Schläuche mit dem Patienten 10 verbunden zu werden. In dem Blutanschlussmodul 22 wird das unbehandelte Blut des Stromes 64 über ein Ventil bzw. eine Schlauchabsperrklemme 182, einen Hämatokritsensor 184, einen arteriellen Drucksensor 186 und eine Blutpumpe 188 dem Strom 66 unbehandelten Bluts zugeführt. Nach Durchlauf des Dialysators 24 gemäß einer bekannten Bauart fließt das nunmehr behandelte Blut in dem Strom 68 zurück zum Blutanschlussmodul 22, wo es über einen venösen Drucksensor 190, einen Luft- und Blutdetektor 192 und ein Ventil bzw. eine Schlauchabsperrklemme 194 in den Strom 70 behandelten Bluts und somit zum Patienten 10 fließt. Des Weiteren kann in dem Blutanschlussmodul 22 Substituat von dem Substituatstrom 60 über eine Substituatpumpe 196 in den Strom 70 behandelten Bluts eingeleitet werden. Um eine Verklumpung des Blut zu verhindern, weist das Blutanschlussmodul 22 eine Antikoagultionspumpe 198 auf, mittels derer ein Antikoagulans stromabwärts der Blutpumpe 188 in das Blut eingeleitet werden kann.

Die Ventile bzw. Schlauchabsperrklemmen 182 und 194 sowie die Pumpen 188, 190, 196 und 198 stellen offenbarungsgemäße Aktoren dar. Die Sensoren 184, 186, 190 und 192 stellen offenbarungsgemäße Sensoren dar. Das Blutanschlussmodul 22 weist eine Modulsteuerungseinheit 200 auf, die über (nicht gezeigte) Leitungen mit den Ventilen 182 und 194, mit den Pumpen 188, 190, 196 und 198 sowie mit den Sensoren 184, 186, 190 und 192 verbunden ist. Des Weiteren ist die Modulsteuerungseinheit 200 über eine der Leitungen 74 mit dem Hauptsteuerungsmodul 26 verbunden. Das Blutanschlussmodul 22 ist als offenbarungsgemäßes Selbststeuerungsmodul ausgebildet. Das Blutanschlussmodul 22 kann die Ventile 182 und 194 und/oder die Pumpen 188, 190, 196 und 198 entsprechend einer Vorgabe von dem Hauptsteuerungsmodul 26 ansteuern und beispielsweise auf Anweisung die Zufuhr von Substituat zum Patienten 10 unterbinden oder die Abnahme von Blut stoppen.

Offenbarungsgemäß sind die Modulsteuerungseinheiten 106, 135, 160, 170, 180 und 200 der Module 12 bis 22 sowie die Überwachungseinheit 138 des Dialysierflüssigkeitsaufbereitungsmoduls 14, wie in Fig. 1 angedeutet, miteinander über Leitungen 202 verbunden. Nachdem die Module 12 bis 22 allesamt als Selbststeuerungsmodule ausgebildet sind, können deren Modulsteuerungseinheiten 106, 135, 160, 170, 180 und 200 vorbestimmte Zustandsänderungen des jeweiligen Moduls 12, 14, 16, 18, 20 und 22 registrieren und untereinander ohne Einbeziehung des Hauptsteuerungsmoduls 4 mittels offenbarungsgemäßer Modulzustandsänderungsmeldungen kommunizieren. Die Modulsteuerungseinheiten 106, 135, 160, 170, 180 und 200 können auch derart ausgebildet sein, dass sie über das Hauptsteuerungsmodul 4 miteinander kommunizieren.

Registriert zum Beispiel einer der Temperatursensoren 132 und 133 des Dialysierflüssigkeitsaufbereitungsmoduls 14, dass die Temperatur des aufbereiteten Wassers von einer vorbestimmten Temperatur abweicht (höher / niedriger als die vorbestimmte Temperatur ist), wird eine entsprechende Modulzustandsänderungsmeldung von der Modulsteuerungseinheit 135 des Dialysierflüssigkeitsaufbereitungsmoduls 14 an die Modulsteuerungseinheit 106 des Wasseraufbereitungsmoduls 12 gesendet, welche daraufhin als eine vorbestimmte Aktion das Heizelement 94 ansteuert (das heißt, die dem Heizelement 94 zugeführte Leistung verringert / erhöht), um die vorbestimmte Temperatur zu erreichen.

Die Modulzustandsänderungsmeldung von der Modulsteuerungseinheit 135 kann parallel zu der Modulsteuerungseinheit 106 des Wasseraufbereitungsmoduls 12 auch zu der Modulsteuerungseinheit 170 des Dialysierflüssigkeitsanschlussmoduls 18 gesendet werden, welche daraufhin als eine vorbestimmte Aktion das Ventil 163 schließt, das Ventil 162 öffnet, die nicht richtig temperierte Dialysierflüssigkeit durch die Bypassleitung 169 ableitet und somit die Zufuhr von der nicht richtig temperierten Dialysierflüssigkeit an den Dialysator 24 unterbindet.

Damit die jeweiligen Modulsteuerungseinheiten Zustandsänderungen in Form von Abweichungen von Messdaten von vorbestimmten Werten beziehungsweise von vorbestimmten Toleranzbereichen registrieren können, sind auf denjenigen Selbststeuerungsmodulen, welche zumindest einen Sensor aufweisen, entsprechende vorbestimmte Werte / Toleranzbereiche für den oder die Sensoren, die zu dem jeweiligen Selbststeuerungsmodul gehören, auf der jeweiligen Modulsteuerungseinheit abgespeichert.

Damit die Modulsteuerungseinheiten passende vorbestimmte Aktionen nach Empfang einer Modulzustandsänderungsmeldung durchführen können, können die Zustandsänderungen kategorisiert sein. Beispielsweise kann eine erste Kategorie von Zustandsänderungen vorgesehen werden, welche ein sofortiges Herunterfahren der einzelnen Selbststeuerungsmodule der Behandlungsvorrichtung 2 erfordert, und kann eine zweite Kategorie vorgesehen werden, welche nur ein Abwarten einzelner Selbststeuerungsmodule erfordert. Auf jeder Modulsteuerungseinheit eines jeden Selbststeuerungsmoduls ist für jede Kategorie eine vorbestimmte Aktion abgespeichert, die bei Eintritt einer Zustandsänderung der entsprechenden Kategorie ausgeführt wird.

Registriert beispielsweise die Überwachungseinheit 138 des Dialysierflüssigkeitsaufbereitungsmoduls 14, dass die Messwerte der Leitfähigkeitssensoren 116 und 118 voneinander abweichen, bedeutet dies, dass die für das Überleben des Patienten 10 hochrelevante Überwachung der Zusammensetzung der Dialysierflüssigkeit fehlerhaft ist. Aufgrund der Relevanz der Überwachung der Zusammensetzung müsste die Behandlung abgebrochen werden und würde diese Zustandsänderung des Dialysierflüssigkeitsaufbereitungsmoduls 14 folglich eine Modulzustandsänderungsmeldung der ersten Kategorie auslösen, worauf sofort die Dialysierflüssigkeit von der Modulsteuerungseinheit 170 des Dialysierflüssigkeitsanschlussmoduls 18 durch die Bypassleitung 169 und das Substituat von der Modulsteuerungseinheit 180 des Hämodiafiltrationsmoduls 20 abgeleitet werden würden, bevor anschließend die gesamte Blutbehandlungsvorrichtung 2 herunter gefahren wird. Auch ist die Überwachungseinheit 138 des Dialysierflüssigkeitsaufbereitungsmoduls 14 derart ausgebildet, dass von ihr eine Modulzustandsänderungsmeldung der ersten Kategorie auslöst wird, wenn die von dem Temperatursensor 133 gemessene Temperatur von der von dem Temperatursensor 134 gemessenen Temperatur abweicht. Auch aufgrund dieser Modulzustandsänderungsmeldung der ersten Kategorie würde die Dialysierflüssigkeit von der Modulsteuerungseinheit 170 des Dialysierflüssigkeitsanschlussmoduls 18 durch die Bypassleitung 169 und das Substituat von der Modulsteuerungseinheit 180 des Hämodiafiltrationsmoduls 20abgeleitet werden, bevor anschließend die gesamte Blutbehandlungsvorrichtung 2 heruntergefahren oder zumindest in einen Standby-Modus gebracht wird.

Weicht beispielsweise nur eine von dem Temperatursensor 96 gemessene Temperatur von einem vorbestimmten Wert ab, kann von der Modulsteuerungseinheit 106 des Wasseraufbereitungsmoduls 12 nur eine Modulzustandsänderungsmeldung der zweiten Kategorie erfolgen. Dementsprechend würden zwar ebenso zunächst von der Modulsteueruungseinheit 170 des Dialysierflüssigkeitsanschlussmoduls 18 die Dialysierflüssigkeit durch die Bypassleitung 169 und von der Modulsteuerungseinheit 180 des Hämodiafiltrationsmoduls 20 das Substituat abgeleitet werden, da aber die Möglichkeit besteht, dass die Temperatur mittels des Heizelements 94 reguliert wird, besteht keine Notwendigkeit, die Blutbehandlungsvorrichtung 2 unmittelbar herunterzufahren.

Die in den Figuren 1 bis 7 gezeigte und voranstehend beschriebene Ausführungsform der erfindungsgemäßen Blutbehandlungsvorrichtung stellt lediglich eine mögliche Umsetzungen der beanspruchten Erfindung dar.

Die Verbindung der Modulsteuerungseinheiten untereinander und jeweils mit dem Hauptsteuerungsmodul müssen nicht wie beschrieben getrennt sein, sondern können mittels eines Bus umgesetzt sein, an welchen sowohl das Hauptsteuerungsmodul, als auch die Modulsteuerungseinheiten der Blutbehandlungsvorrichtung angeschlossen sind. Auch kann die Kommunikation der Modulsteuerungseinheiten untereinander kabellos erfolgen.

### Bezugszeichenliste

- 2: Blutbehandlungsvorrichtung
- 4: Stromversorgung
- 6: Wasserver- und entsorgung
- 8: zentrale Konzentratversorgung
- 10: Patient
- 12: Wasseraufbereitungsmodul
- 14: Dialysierflüssigkeitsaufbereitungsmodul
- 16: Bilanzierungsmodul
- 18: Dialysierflüssigkeitsanschlussmodul
- 20: Hämodiafiltrationsmodul
- 22: Blutanschlussmodul
- 24: Dialysator
- 26: Hauptsteuerungsmodul
- 28: Frischwasserstrom
- 30: Abwasserstrom
- 32: Hauptstrom aufbereiteten Wassers
- 34: Nebenstrom aufbereiteten Wassers
- 36, 50, 56: Dialysatstrom
- 38: basischer Konzentratstrom aus Kanister
- 40: saurer Konzentratstrom aus Kanister
- 42: saurer Konzentratstrom aus zentraler Konzentratversorgung
- 44, 48, 52, 54: Dialysierflüssigkeitsstrom
- 58: Strom überschüssigen Substituats
- 60: Substituatstrom
- 64, 66: Strom unbehandelten Bluts
- 68, 70: Strom behandelten Bluts
- 72: elektrische Leitung
- 74: Leitungen zur Strom- und Datenübertragung
- 76: berührungsempfindlicher Bildschirm
- 78: Eingabetaste
- 80: Vorlaufbehälter
- 82, 100, 102: Ventil des Wasseraufbereitungsmoduls
- 84: Füllstand des Vorlaufbehälters
- 86: Pumpe des Wasseraufbereitungsmoduls
- 88: Drucksensor des Wasseraufbereitungsmoduls
- 90: Entgasungskammer
- 92: Wärmetauscher
- 94: Heizelement
- 96: Temperatursensor des Wasseraufbereitungsmoduls
- 106: Modulsteuerungseinheit des Wasseraufbereitungsmoduls
- 108, 112: Mischkammer
- 110: Kartusche
- 111, 113: Pumpe des Dialysierflüssigkeitsaufbereitungsmoduls
- 114, 116, 118: Leitfähigkeitssensor
- 120 bis 130: Ventil des Dialysierflüssigkeitsaufbereitungsmoduls
- 132, 133, 134: Temperatursensor des Dialysierflüssigkeitsaufbereitungsmoduls
- 135: Modulsteuerungseinheit des Dialysierflüssigkeitsaufbereitungsmoduls
- 136: Ausführungseinheit
- 138: Überwachungseinheit
- 140: Dialysierflüssigkeitspumpe
- 142, 144: Bilanzkammer
- 146: Dialysierflüssigkeitsfilter
- 148: Dialysatpumpe
- 150: Ultrafiltratpumpe
- 152 bis 158: Ventil des Bilanzierungsmoduls
- 160: Modulsteuerungseinheit des Bilanzierungsmoduls
- 162, 163, 164: Ventil des Dialysierflüssigkeitsanschlussmoduls
- 165: Blutleckdetektor des Dialysierflüssigkeitsanschlussmoduls
- 166: UV-Sensor des Dialysierflüssigkeitsanschlussmoduls
- 167: Temperatursensor des Dialysierflüssigkeitsanschlussmoduls
- 168: Leitfähigkeitssensor des Dialysierflüssigkeitsanschlussmoduls
- 169: Bypassleitung
- 170: Modulsteuerungseinheit des Dialysierflüssigkeitsanschlussmoduls
- 172: Hämodiafiltrationsfilter
- 174, 176, 178: Ventil des Hämodiafiltrationsmoduls
- 180: Modulsteuerungseinheit des Hämodiafiltrationsmoduls
- 182, 194: Ventil des Blutanschlussmoduls
- 184: Hämatokritsensor des Blutanschlussmoduls
- 186: arterieller Drucksensor des Blutanschlussmoduls
- 188: Blutpumpe des Blutanschlussmoduls
- 190: venöser Drucksensor des Blutanschlussmoduls
- 192: Luft-und Blutdetektor des Blutanschlussmoduls
- 196: Substituatpumpe des Blutanschlussmoduls
- 200: Modulsteuerungseinheit des Blutanschlussmoduls
- 202: Leitung zwischen Modulsteuerungseinheiten

## Patentansprüche

1. Modulare Blutbehandlungsvorrichtung (2) mit
mehreren Modulen (12, 14, 16, 18, 20, 22), die ausgebildet sind, zur Durchführung einer Blutbehandlung an einem Patienten (10) lösbar miteinander gekoppelt zu werden,
einer Hauptsteuerungseinheit (26), die ausgebildet ist, die miteinander gekoppelten Module (12, 14, 16, 18, 20, 22) zur Durchführung der Blutbehandlung zu steuern,
Sensoren (88, 96; 114, 116, 132, 133, 165, 167, 168; 186,190, 192) und
Aktoren (82, 86, 94, 100, 102; 111, 113, 120-130; 140, 148, 150, 152-158; 162-164; 174-178; 182, 194, 196), wobei
zumindest zwei der Module (12, 14, 16, 18, 20, 22) als Selbststeuerungsmodule ausgebildet sind, die jeweils als Komponenten zumindest einen der Aktoren (82, 86, 94, 100, 102; 111, 113, 120-130; 140, 148, 150, 152-158; 162-164; 174-178; 182, 194, 196) und zumindest eine Modulsteuerungseinheit (106, 135, 160, 170, 180,200) aufweisen, und
die Modulsteuerungseinheiten (106, 135, 160, 170, 180, 200) ausgebildet sind,
den zumindest einen Aktor (82, 86, 94, 100, 102; 111, 113, 120-130; 140, 148, 150, 152-158; 162-164; 174-178; 182, 194, 196) desselben Selbststeuerungsmoduls steuern zu können,
eine vorbestimmte Zustandsänderung des zur jeweiligen Modulsteuerungseinheit (106, 135, 160, 170, 180, 200) gehörigen Selbststeuerungsmoduls (12, 14, 16, 18, 20, 22) feststellen zu können,
bei Feststellung der vorbestimmten Zustandsänderung eine entsprechende Modulzustandsänderungsmeldung direkt zu der zumindest einen anderen Modulsteuerungseinheit (106, 135, 160, 170, 180, 200) senden zu können,
eine Modulzustandsänderungsmeldung direkt von der zumindest einen anderen Modulsteuerungseinheit (106, 135, 160, 170, 180, 200) empfangen zu können und
bei Empfang einer Modulzustandsänderungsmeldung von der zumindest einen anderen Modulsteuerungseinheit den zumindest einen Aktor (82, 86, 94, 100, 102; 111, 113, 120-130; 140, 148, 150, 152-158; 162-164; 174-178; 182, 194, 196) desselben Selbststeuerungsmoduls (12, 14, 16, 18, 20, 22) anzuweisen, eine vorbestimmte Aktion auszuführen.

2. Blutbehandlungsvorrichtung (2) nach Anspruch 1, **dadurch gekennzeichnet, dass** die von zumindest einer der Modulsteuerungseinheiten (106, 135, 160, 170, 180, 200) angewiesene vorbestimmte Aktion ein Schalten des zumindest einen Aktors (82, 86, 94, 100, 102; 111, 113, 120-130; 140, 148, 150, 152-158; 162-164; 174-178; 182, 194, 196) desselben Selbststeuerungsmoduls (12, 14, 16, 18, 20, 22) in eine Nullstellung und/oder ein Freischalten einer Bypassleitung (169) desselben Selbststeuerungsmoduls für ein Fluidsystem der Blutbehandlungsvorrichtung (2) ist.

3. Blutbehandlungsvorrichtung (2) nach Anspruch 1 oder 2, **dadurch gekennzeichnet, dass**
zumindest eines der Selbststeuerungsmodule (14) zumindest einen der Sensoren (116, 118) aufweist und über den zumindest einen Sensor (116; 133) hinaus als weitere Komponente einen Überwachungssensor (118; 134) aufweist,
die Modulsteuerungseinheit (135) des Selbststeuerungsmoduls (14) mit dem Überwachungssensor (118; 134) eine Ausführungseinheit (136) und eine Überwachungseinheit (138) aufweist,
der zumindest eine Sensor (116; 133) des Selbststeuerungsmoduls (14) mit der Ausführungseinheit 136) verbunden ist,
der Überwachungssensor (118; 134) mit der Überwachungseinheit (138) verbunden ist und
die Modulsteuerungseinheit (135) ausgebildet ist, die vorbestimmte Modulzustandsänderung des zugehörigen Selbststeuerungsmoduls (14) durch Abgleich von Daten der Ausführungseinheit (136) und der Überwachungseinheit (138) zu erkennen.

4. Blutbehandlungsvorrichtung (2) nach Anspruch 3, **dadurch gekennzeichnet, dass** die Überwachungseinheit (138) auch mit dem zumindest einen Sensor (116) verbunden ist.

5. Blutbehandlungsvorrichtung (2) nach Anspruch 3 oder 4, **dadurch gekennzeichnet, dass** sich die Bauart des zumindest einen Sensors (116) und die Bauart des Überwachungssensors (118) des Selbststeuerungsmoduls (14) unterscheiden und/oder sich die Bauart eines Mikroprozessors der Ausführungseinheit (136) und die Bauart eines Mikroprozessors der Überwachungseinheit (138) unterscheiden.

6. Blutbehandlungsvorrichtung (2) nach einem der Ansprüche 1 bis 5, **dadurch gekennzeichnet, dass** zumindest eines der Selbststeuerungsmodule (12, 14, 16, 18, 20, 22) Kalibrierdaten für zumindest einen Teil der Komponenten desselben Selbststeuerungsmoduls (12, 14, 16, 18, 20, 22) speichert.

7. Blutbehandlungsvorrichtung (2) nach einem der Ansprüche 1 bis 6, **dadurch gekennzeichnet, dass**
zumindest eines der Selbststeuerungsmodule (12, 14, 16, 18, 20, 22) zumindest einen Schwellenwert für zumindest einen Teil der Komponenten desselben Selbststeuerungsmoduls (12, 14, 16, 18, 20, 22) speichert und
die Modulsteuerungseinheit (106, 135, 160, 170, 180, 200) desselben Selbststeuerungsmoduls (12, 14, 16, 18, 20, 22) ausgebildet ist, die vorbestimmte Modulzustandsänderung des zugehörigen Selbststeuerungsmoduls (12, 14, 16, 18, 20, 22) durch Abgleich von Daten zumindest einer der Komponenten desselben Selbststeuerungsmoduls (12, 14, 16, 18, 20, 22) und dem zumindest einen Schwellenwert zu erkennen.

8. Blutbehandlungsvorrichtung (2) nach einem der Ansprüche 1 bis 7, **dadurch gekennzeichnet, dass**
die Hauptsteuerungseinheit als eigenes Modul in Form eines Hauptsteuerungsmoduls (26) ausgebildet ist,
die Blutbehandlungsvorrichtung (2) die zur Durchführung einer Blutbehandlung eines Patienten notwendigen Module (12, 14, 16, 18, 20, 22) mehrfach aufweist, so dass die Blutbehandlungsvorrichtung (2) zur gleichzeitigen Blutbehandlung mehrerer Patienten geeignet ist, und
das Hauptsteuerungsmodul (26) ausgebildet ist, die gleichzeitige Blutbehandlung der mehreren Patienten steuern zu können.

9. Blutbehandlungsvorrichtung (2) nach einem der Ansprüche 1 bis 8, **gekennzeichnet, durch** ein Bedien- und Anzeigemodul, das ausgebildet ist, Daten zumindest einzelner Module (12, 14, 16, 18, 20, 22) der Behandlungsvorrichtung (2) anzuzeigen, Befehle von einem Benutzer zu empfangen und die Befehle an die Hauptsteuerungseinheit und/oder direkt an zumindest einzelne Module (12, 14, 16, 18, 20, 22) der Behandlungsvorrichtung (2) weiterzuleiten.

10. Blutbehandlungsvorrichtung (2) nach einem der Ansprüche 1 bis 9, **gekennzeichnet, durch** ein Kalibriermodul, das zumindest ein Referenzmessgerät aufweist, welches ausgebildet ist, einen Abgleich mit zumindest einem der Sensoren zu ermöglichen.

## Claims

1. A modular blood treatment device (2) comprising
a plurality of modules (12, 14, 16, 18, 20, 22) configured to be detachably coupled together to perform blood treatment on a patient (10),
a main control unit (26) configured to control the coupled modules (12, 14, 16, 18, 20, 22) to perform the blood treatment,
sensors (88, 96; 114, 116, 132, 133, 165, 167, 168; 186, 190, 192), and actuators (82, 86, 94, 100, 102; 111, 113, 120-130; 140, 148, 150, 152-158; 162-164; 174-178; 182, 194, 196), wherein
at least two of the modules (12, 14, 16, 18, 20, 22) are configured as self-control modules, each of which includes, as components, at least one of the actuators (82, 86, 94, 100, 102; 111, 113, 120-130; 140, 148, 150, 152-158; 162-164; 174-178; 182, 194, 196) and at least one module control unit (106, 135, 160, 170, 180, 200), and
the module control units (106, 135, 160, 170, 180, 200) are configured to
be able to control the at least one actuator (82, 86, 94, 100, 102; 111, 113, 120-130; 140, 148, 150, 152-158; 162-164; 174-178; 182, 194, 196) of the same self-control module,
be able to detect a predetermined state change of the self-control module (12, 14, 16, 18, 20, 22) associated with the respective module control unit (106, 135, 160, 170, 180, 200),
be able, upon detection of the predetermined state change, to send a corresponding module state change message directly to the at least one other module control unit (106, 135, 160, 170, 180, 200), and
upon receiving a module state change message from the at least one other module control unit, to control the at least one actuator (82, 86, 94, 100, 102; 111, 113, 120-130; 140, 148, 150, 152-158; 162-164; 174-178; 182, 194, 196) of the same self-control module (12, 14, 16, 18, 20, 22) to perform a predetermined action.

2. The blood treatment device (2) according to claim 1, **characterized in that** the predetermined action commanded by at least one of the module control units (106, 135, 160, 170, 180, 200) is to activate the at least one actuator (82, 86, 94, 100, 102; 111, 113, 120-130; 140, 148, 150, 152-158; 162-164; 174-178; 182, 194, 196) of the same self-control module (12, 14, 16, 18, 20, 22) to a zero position and/or activating a bypass line (169) of the same self-control module for a fluid system of the blood treatment device (2).

3. The blood treatment device (2) according to claim 1 or 2, **characterized in that**
at least one of the self-control modules (14) comprises at least one of the sensors (116, 118) and, in addition to said at least one sensor (116; 133), includes a monitoring sensor (118; 134) as a further component,
the module control unit (135) of the self-control module (14) comprises, in conjunction with the monitoring sensor (118; 134) an execution unit (136) and a monitoring unit (138),
the at least one sensor (116; 133) of the self-control module (14) is connected to the execution unit (136),
the monitoring sensor (118; 134) is connected to the monitoring unit (138), and
the module control unit (135) is configured to detect the predetermined module state change of the associated self-control module (14) by comparing data from the execution unit (136) and the monitoring unit (138).

4. The blood treatment device (2) according to claim 3, **characterized in that** the monitoring unit (138) is also connected to the at least one sensor (116).

5. The blood treatment device (2) according to claim 3 or 4, **characterized in that** the design of the at least one sensor (116) and the design of the monitoring sensor (118) of the self-control module (14) differ and/or the design of a microprocessor of the execution unit (136) and the design of a microprocessor of the monitoring unit (138) differ.

6. The blood treatment device (2) according to any one of claims 1 through 5, **characterized in that** at least one of the self-control modules (12, 14, 16, 18, 20, 22) stores calibration data for at least part of the components of the same self-control module (12, 14, 16, 18, 20, 22).

7. The blood treatment device (2) according to any one of claims 1 through 6, **characterized in that**
at least one of the self-control modules (12, 14, 16, 18, 20, 22) stores at least one threshold value for at least part of the components of the same self-control module (12, 14, 16, 18, 20, 22) and
the module control unit (106, 135, 160, 170, 180, 200) of the same self-control module (12, 14, 16, 18, 20, 22) is configured to detect the predetermined module state change of the associated self-control module (12, 14, 16, 18, 20, 22) by comparing data from at least one of the components of the same self-control module (12, 14, 16, 18, 20, 22) with the at least one threshold value.

8. The blood treatment device (2) according to any one of claims 1 through 7, **characterized in that**
the main control unit is configured as a separate module in the form of a main control module (26),
the blood treatment device (2) includes the modules (12, 14, 16, 18, 20, 22) necessary to perform blood treatment on a patient multiple times, such that the blood treatment device (2) is suitable for the simultaneous blood treatment of multiple patients, and
the main control module (26) is configured to be able to control the simultaneous blood treatment of the multiple patients.

9. The blood treatment device (2) according to any one of claims 1 through 8, **characterized by** a control and display module configured to display data from at least individual modules (12, 14, 16, 18, 20, 22) of the treatment device (2), to receive commands from a user, and to forward the commands to the main control unit and/or directly to at least individual modules (12, 14, 16, 18, 20, 22) of the treatment device (2).

10. The blood treatment device (2) according to any one of claims 1 through 9, **characterized by** a calibration module including at least one reference measuring device configured to enable calibration with at least one of the sensors.

## Revendications

1. Dispositif de traitement du sang (2) modulaire avec
plusieurs modules (12, 14, 16, 18, 20, 22) qui sont configurés afin d'être couplés entre eux de manière amovible pour la réalisation d'un traitement sanguin,
une unité de commande principale (26) qui est configurée afin de commander les modules (12, 14, 16, 18, 20, 22) couplés entre eux pour la réalisation du traitement sanguin,
des capteurs (88, 96 ; 114, 116, 132, 133, 165, 167, 168 ; 186, 190, 192) et
des actionneurs (82, 86, 94, 100, 102 ; 111, 113, 120-130 ; 140, 148, 150, 152-158 ; 162-164 ; 174-178 ; 182, 194, 196), dans lequel
au moins deux des modules (12, 14, 16, 18, 20, 22) sont configurés comme des modules de commande automatique qui présentent respectivement comme composants au moins l'un des actionneurs (82, 86, 94, 100, 102 ; 111, 113, 120-130 ; 140, 148, 150, 152-158 ; 162-164 ; 174-178 ; 182, 194, 196) et au moins une unité de commande de module (106, 135, 160, 170, 180, 200) et
les unités de commande de module (106, 135, 160, 170, 180, 200) sont configurées
afin de pouvoir commander le au moins un actionneur (82, 86, 94, 100, 102 ; 111, 113, 120-130 ; 140, 148, 150, 152-158 ; 162-164 ; 174-178 ; 182, 194, 196) du même module de commande automatique,
afin de pouvoir constater une modification d'état prédéterminée du module de commande automatique (12, 14, 16, 18, 20, 22) appartenant à l'unité de commande de module (106, 135, 160, 170, 180, 200) respective,
afin de pouvoir envoyer un message de modification d'état de module correspondant directement à la au moins une autre unité de commande de module (106, 135, 160, 170, 180, 200) lors de la constatation de la modification d'état prédéterminée,
afin de pouvoir recevoir un message de modification d'état de module directement de la au moins une autre unité de commande de module (106, 135, 160, 170, 180, 200) et
afin d'ordonner le au moins un actionneur (82, 86, 94, 100, 102 ; 111, 113, 120-130 ; 140, 148, 150, 152-158 ; 162-164 ; 174-178 ; 182, 194, 196) du même module de commande automatique (12, 14, 16, 18, 20, 22) d'exécuter une action prédéterminée lors de la réception d'un message de modification d'état de module de l'au moins une autre unité de commande de module.

2. Dispositif de traitement du sang (2) selon la revendication 1, **caractérisé en ce que** l'action prédéterminée ordonnée par la au moins une des unités de commande de module (106, 135, 160, 170, 180, 200) est une commutation de l'au moins un actionneur (82, 86, 94, 100, 102 ; 111, 113, 120-130 ; 140, 148, 150, 152-158 ; 162-164 ; 174-178 ; 182, 194, 196) du même module de commande automatique (12, 14, 16, 18, 20, 22) dans une position nulle et/ou un déverrouillage d'une conduite de dérivation (169) du même module de commande automatique pour un système fluidique du dispositif de traitement du sang (2).

3. Dispositif de traitement du sang (2) selon la revendication 1 ou 2, **caractérisé en ce que**
au moins l'un des modules de commande automatique (14) présente au moins l'un des capteurs (116, 118) et présente un capteur de surveillance (118 ; 134) en plus de l'au moins un capteur (116 ; 133) comme composant supplémentaire,
l'unité de commande de module (135) du module de commande automatique (14) avec le capteur de surveillance (118 ; 134) présente une unité d'exécution (136) et une unité de surveillance (138),
le au moins un capteur (116 ; 133) du module de commande automatique (14) est relié à l'unité d'exécution (136),
le capteur de surveillance (118 ; 134) est relié à l'unité de surveillance (138) et
l'unité de commande de module (135) est configurée afin de détecter la modification d'état de module prédéterminée du module de commande automatique (14) afférent par comparaison de données de l'unité d'exécution (136) et de l'unité de surveillance (138).

4. Dispositif de traitement du sang (2) selon la revendication 3, **caractérisé en ce que** l'unité de surveillance (138) est aussi reliée à l'au moins un capteur (116).

5. Dispositif de traitement du sang (2) selon la revendication 3 ou 4, **caractérisé en ce que** le type de l'au moins un capteur (116) et le type du capteur de surveillance (118) du module de commande automatique (14) diffèrent et/ou le type d'un microprocesseur de l'unité d'exécution (136) et le type d'un microprocesseur de l'unité de surveillance (138) diffèrent.

6. Dispositif de traitement du sang (2) selon l'une quelconque des revendications 1 à 5, **caractérisé en ce qu'**au moins l'un des modules de commande automatique (12, 14, 16, 18, 20, 22) enregistre des données d'étalonnage pour au moins une partie des composants du même module de commande automatique (12, 14, 16, 18, 20, 22).

7. Dispositif de traitement du sang (2) selon l'une quelconque des revendications 1 à 6, **caractérisé en ce que**
au moins l'un des modules de commande automatique (12, 14, 16, 18, 20, 22) enregistre au moins une valeur seuil pour au moins une partie des composants du même module de commande automatique (12, 14, 16, 18, 20, 22) et
l'unité de commande de module (106, 135, 160, 170, 180, 200) du même module de commande automatique (12, 14, 16, 18, 20, 22) est configurée afin de détecter la modification d'état de module prédéterminée du module de commande automatique (12, 14, 16, 18, 20, 22) afférent par comparaison de données au moins d'un des composants du même module de commande automatique (12, 14, 16, 18, 20, 22) et de la au moins une valeur seuil.

8. Dispositif de traitement du sang (2) selon l'une quelconque des revendications 1 à 7, **caractérisé en ce que**
l'unité de commande principale est configurée comme propre module sous la forme d'un module de commande principal (26),
le dispositif de traitement du sang (2) présente plusieurs fois les modules (12, 14, 16, 18, 20, 22) nécessaires à la réalisation d'un traitement du sang d'un patient, de sorte que le dispositif de traitement du sang (2) soit approprié au traitement du sang simultané de plusieurs patients et
le module de commande principal (26) est configuré afin de pouvoir commander le traitement du sang simultané de plusieurs patients.

9. Dispositif de traitement du sang (2) selon l'une quelconque des revendications 1 à 8, **caractérisé par** un module de commande et d'affichage qui est configuré afin d'afficher des données au moins de modules individuels (12, 14, 16, 18, 20, 22) du dispositif de traitement (2), de recevoir des ordres d'un utilisateur et de transmettre les ordres à l'unité de commande principale et/ou directement à au moins des modules individuels (12, 14, 16, 18, 20, 22) du dispositif de traitement (2).

10. Dispositif de traitement du sang (2) selon l'une quelconque des revendications 1 à 9, **caractérisé par** un module d'étalonnage qui présente au moins un appareil de mesure de référence qui est configuré afin de permettre une comparaison avec au moins l'un des capteurs.
